Europäisches Patentamt

European Patent Office

Office européen des brevets

(19)

(11) Veröffentlichungsnummer : **0 440 074 B1**

(12) **EUROPÄISCHE PATENTSCHRIFT**

(45) Veröffentlichungstag der Patentschrift :
06.04.94 Patentblatt 94/14

(51) Int. Cl.$^5$ : **C12P 19/00, C12P 19/04, C12P 19/14, C07H 3/06**

(21) Anmeldenummer : 91100776.3

(22) Anmeldetag : 22.01.91

(54) **Verfahren zur Herstellung eines glucose-, fructose- und saccharosearmen Inulooligosaccharid-Produktes.**

Die Akte enthält technische Angaben, die nach dem Eingang der Anmeldung eingereicht wurden und die nicht in dieser Patentschrift enthalten sind.

(30) Priorität : 02.02.90 DE 4003140

(43) Veröffentlichungstag der Anmeldung :
07.08.91 Patentblatt 91/32

(45) Bekanntmachung des Hinweises auf die Patenterteilung :
06.04.94 Patentblatt 94/14

(84) Benannte Vertragsstaaten :
AT BE CH DE DK ES FR GB GR IT LI LU NL SE

(56) Entgegenhaltungen :
EP-A- 171 026
EP-A- 188 047

(56) Entgegenhaltungen :
EP-A- 293 935
PATENT ABSTRACTS OF JAPAN, unexamined applications, C field, Band 12, Nr. 96, 29 März 1988, S. 115 C 484 Kokai Nr. 62-228 293

(73) Patentinhaber : SÜDZUCKER AKTIENGESELLSCHAFT MANNHEIM/OCHSENFURT
Maximilianstrasse 10
D-68165 Mannheim (DE)

(72) Erfinder : Heinz, Fritz, Prof.-Dr.
Emscher Weg 9
W-3000 Hannover 61 (DE)
Erfinder : Vogel, Manfred, Dr.
Am Höllpfad
W-6719 Neuleiningen (DE)

(74) Vertreter : Gleiss, Alf-Olav, Dipl.-Ing. et al
Gleiss & Grosse Patentanwaltskanzlei
Maybachstrasse 6A
D-70469 Stuttgart (DE)

EP 0 440 074 B1

## Beschreibung

Die Erfindung betrifft ein Verfahren zur Herstellung eines glucose-, fructose- und saccharosearmen Inulooligosaccharid-Produktes, wobei von inulinhaltigem Pflanzenmaterial ausgegangen wird.

Inulin ist ein Polysaccharid und gehört zur Gruppe der Fructane. Es kommt in den verschiedensten Pflanzen vor, hauptsächlich aber in der Pflanzenfamilie der Compositen, wo es als Reservepolysaccharid vorliegt. Hohe Gehalte an Inulin sind in Topinambur- und Dahlienknollen sowie in Zichorienwurzeln zu finden.

Die Kettenlänge von Inulin hängt sowohl von der Wachstumsphase der Pflanzen als auch vom pflanzlichen Ursprung ab. Es ist ein Heterofructan, da es an einer $\beta$-2-1-verknüpften Kette von Fructofuranosemolekülen als Abschluß eine $\alpha$-D-Glucose am reduzierenden Ende trägt.

Inulin ist in kaltem Wasser nicht löslich, läßt sich aber in der Wärme aus vorzerkleinertem Pflanzenmaterial, ähnlich wie bei der Extraktion von Saccharose aus Zuckerrüben beschrieben, extrahieren.

Des weiteren sind eine Reihe von Anwendungsmöglichkeiten für Inulin bekannt. So beschreiben R.H.F. Beck und W. Praznik (Stärke 38, 11, 391 - 394, 1986), daß es über die unmittelbare Verwendung als solches hinaus auch als Ausgangspunkt zur Darstellung von D-Fructose oder von Inulooligosacchariden dienen kann. Daneben stellt es nach Hydrolyse auch eine gute Kohlenstoffquelle für Fermentationszwecke dar.

Es ist aus der Literatur bekannt (J.P. Guiraud und P. Galzy, Industries Alimentaires et Agricoles 98, 45, 1981), daß Inulin durch Zugabe von Säure unter relativ milden Bedingungen, z. B. pH 1 - 2, bei 80 - 100 °C für 1 - 2 Stunden teilweise hydrolysiert wird. Hierbei entstehen aber durch die Zerstörung der Fructose unerwünschte Nebenprodukte.

In JP-61-40754 wird ausgehend von Inulin durch Zugabe von Oxalsäure ein Inulooligosaccharid-Gemisch erhalten, das hauptsächlich kurzkettige Oligosaccharide, wie Inulobiose und Inulotriose enthält. Die Oligosaccharide mit längerer Kettenlänge sind hingegen nur zu geringem Anteil vorhanden.

Beschrieben wird auch die enzymatische Hydrolyse von Inulin. Die als Inulinasen bekannten Enzyme sind mikrobiellen Ursprungs und werden vorzugsweise von Hefen, wie Kluyveromyces- und Candida-Stämmen produziert. Bekannt sind auch pilzliche Inulinasen. Hier sind hauptsächlich Aspergillus-, Fusarium- und Penicillium-Stämme zu nennen.

In allen Fällen setzen sich die Inulinasen aus Exo- und Endo-Inulinasen zusammen, wobei letztere aus Inulin bevorzugt die entsprechenden Oligosaccharide bilden (L. Zittan, Stärke 33, 11, 373 - 377, 1981).

S. Pedersen und B.E. Norman (Vortrag: Chemical Aspects of Food Enzymes, Symposium in Reading, England, 16./17.09.1986) berichten über die Wirkung einer Endo-Inulinase, isoliert aus Aspergillus ficum. Die partielle Hydrolyse des Inulins erfolgte mit 20 Gew.% Inulin bei 60 °C, pH 4,5 für 6 Stunden.

Im EP-A0293935 wird die Herstellung eines Futterzusatzes beschrieben, in dem Zichorien nach Zerkleinerung und Trocknung bei 180 °C direkt als Futterzusatz Verwendung finden oder nach Zugabe von Wasser und Endo-Inulinase in Inulooligosaccharide gespalten werden, welche einen hohen Gehalt von Sacchariden in einem Bereich von F2 bis F6 besitzen. Diese Produkte werden dem Tierfutter, insbesondere dem Futter für Jungtiere zugesetzt, welche unter Diarrhöe leiden. Die Inuline haben dabei den Effekt, daß sie durch die tierischen Enzyme nicht zersetzt und daher vom Darm nicht absorbiert werden, so daß sie im Dickdarm als Nahrungsquelle das Wachstum von Lactobazillen fördern. Die die Diarrhöe verursachenden Colibakterien andererseits können die Inuliene nicht verwerten, so daß sie sich ihrerseits nicht vermehren. Bei diesem Mechanismus ist es uninteressant, ob die verabreichten Mittel neben den Inulinen noch Mono- und Disaccharide oder zusätzliche Oligosaccharide enthalten, die als Nahrungsmittel verwertet werden, so daß gemäß der Tabelle auf Seite 5 etwa 5 % Mono- und Disaccharide und 50 % höhere Saccharide enthalten sind, neben nur 15 % der eigentlich interessierenden Inuline. Für die Verwendung als kalorienreduzierte Süßungsmittel, insbesondere für Diabetiker, sind solche Produkte aber nicht geeignet.

Überdies ist bekannt, daß Frucooligosaccharid-Gemische erhalten werden, wenn Saccharose-Lösungen in Gegenwart einer Fructosyltransferase umgesetzt werden (DE-PS- 3112842). Dabei wird das Fructosemolekül der Saccharose auf ein Akzeptormolekül Saccharose übertragen. Erhalten wird eine Produktlösung, die z. B. 26 % 1-Kestose, 26 % Nystose, 5 % Fructosyl-Nystose sowie 32 % Monosaccharide, hauptsächlich Glucose und 11 % Saccharose enthält. Diese als Neosugar G bezeichnete Produktlösung wird in der Regel weiteren Reinigungsschritten unterzogen, um den hohen Anteil an Glucose und Saccharose zu reduzieren. Dieses verteuert den Prozess und bedeutet, bezogen auf eingesetzte Saccharose, eine schlechte Produktausbeute.

Es stellte sich daher die Aufgabe, Inulooligosaccharid-Gemische mit geringen Gehalten an Glucose, Fructose und Saccharose mit hoher Ausbeute aus inulinhaltigem Pflanzenmaterial herzustellen. Diese Aufgabe wird durch die im Hauptanspruch wiedergegebenen Maßnahmen gelöst und durch die Maßnahmen der Unteransprüche gefördert.

Im einzelnen ist dazu folgendes zu bemerken:

Das in kleine Stückchen vorzerkleinerte inulinhaltige Pflanzenmaterial wird nach Zugabe von 1 bis 2 Gewichtsteilen Wasser in einem kontinuierlichen Prozeß zellvermahlen, in Sekundenschnelle auf 90 bis 98 °C erwärmt und somit in einen Brei überführt. Die Temperatur wird dabei für 20 bis 60 Minuten auf 90 bis 98 °C belassen, um einerseits die in dem inulinhaltigen Pflanzenmaterial befindlichen Enzyme, wie Invertasen und Phenoloxidasen, zu inaktivieren und andererseits eine Pasteurisierung zu erzielen.

Nach Abkühlung des Breies auf 50 bis 60 °C, insbesondere auf 56 °C, wird mit 33 %iger $H_2SO_4$ ein pH-Wert von 5,0 bis 5,6 unter Rühren eingestellt. Dieser zu anderen Veröffentlichungen relativ hohe pH-Wert ergibt erfindungsgemäß höhere Gehalte an Inulooligosacchariden und vermindert die Gehalte an Monosacchariden. Pro Gramm Inulin werden 1,8 bis 2,4 Enzymeinheiten an Endo-Inulinase zugefügt.

Die Endo-Inulinase läßt man bevorzugt für 13 bis 18 Stunden bei 56 °C unter kontinuierlichem Rühren des Breies einwirken. Dabei wird der pH-Wert zwischen 5,3 bis 5,4 durch Zugabe von z. B. Calziumhydroxid $Ca(OH)_2$ in Form von Kalkmilch konstant gehalten.

Gestoppt wird die enzymatische Reaktion durch Erhöhung des pH-Wertes. Es wird dazu unter schnellem Rühren noch soviel an z. B. Kalkmilch zugegeben, bis sich ein pH-Wert von 10,7 bis 11,0 eingestellt hat. Die Anhebung des pH-Wertes führt zu einer irreversiblen Inaktivierung der Endo-Inulinase und zu einer Flockung löslicher, hoch polymerer Bestandteile des Breies, so daß sich die anschließende Filtration problemlos durchführen läßt.

Nach einer Standzeit des Breies von 30 Minuten erfolgt die Filtration mittels Filterkammerpresse bei einer Temperatur von 50 - 60 °C. Um eine gute Ausbeute an Inulooligosacchariden zu erhalten, wird der Filterkuchen mit Wasser gewaschen. Durch Einleiten von Kohlensäure in die erhaltene inulooligosaccharidhaltige Lösung erfolgt eine Neutralisation.

Die so erhaltene Lösung enthält zwischen 82 - 86 % Inulooligosaccharide, auf Kohlenhydrate bezogen.

Diese Lösung wird nach einer evtl. notwendigen Konzentrierung auf einen TS-Gehalt von 15 - 20 % gebracht und an einem schwach sauren Kationenaustauscher, gefolgt von einem mittelbasischen Anionenaustauscher entsalzt und weitgehend entfärbt. Eine endgültige Entfärbung wird durch Behandlung mit gekörnter Aktivkohle erzielt. Danach wird die Lösung sterilfiltriert und weiter auf einen TS-Gehalt von 75 % aufkonzentriert und in dieser Form als Sirup gelagert. Alternativ dazu ist das Überführen der Inulooligosaaccharide in feste Form, z. B. durch Sprühtrocknung bzw. Schaummattentrocknung möglich.

Die Erfindung betrifft weiter die Aufreinigung der Inulooligosaccharide auf chromatographischem Wege. Dazu wird der filtrierte, auf einen TS-Gehalt von 40 - 60 % gebrachte Sirup am Ca-beladenen Kationenaustauscher bei 60 bis 70 °C in Fraktionen getrennt. Dabei wird weitgehend bei Bedingungen getrennt, wie sie für die Trennung von Glucose/Fructose, ausgehend von Invertzucker, bekannt sind. Durch die chromatographische Trennung werden hochmolekulare Anteile sowie Asche und farbige Komponenten neben freier Glucose und Fructose von den interessierenden Inulooligosacchariden getrennt.

Als Kationenaustauscher kommen solche in Betracht, deren Grundgerüst aus Polystyrolsulfonsäure besteht, die mit 3 bis 6 % Divinylbenzol vernetzt in der Ca-Form vorliegen, wie z. B. Lewatit® TSW40 der Fa. Bayer oder Duolite® C204F der Fa. Rohm & Haas.

Die inulooligosacchridhaltigen Fraktionen werden vereint und anschließend auf einen Trockensubstanzgehalt von 15 bis 20 % eingeengt, filtriert und, wie oben bereits beschrieben, an Kationen- und Anionenaustauschern vollentsalzt und entfärbt.

Dazu hat es sich als vorteilhaft erwiesen, die Lösung zuerst über eine Säule zu geben, die mit einem schwach sauren Kationenaustauscher in der $H^+$-Form gefüllt ist, und anschließend über einen schwach oder mittelbasischen Anionenaustauscher in der $OH^-$-Form. Diese Schritte werden bevorzugt bei Zimmertemperatur durchgeführt.

Die farblosen Effluate, die die Inulooligosaccharide enthalten, werden vereint, auf 15 - 30 % Trockensubstanz aufkonzentriert, steril filtriert, weiter auf einen TS-Gehalt von 75 % aufkonzentriert und in dieser Form als Sirup gelagert. Alternativ dazu ist das Überführen der Inulooligosaccharide in feste Form, z. B. durch Sprühtrocknung bzw. Schaummattentrocknung möglich.

Das so erhaltene Produkt besteht zu 92 bis 94 %, auf Trockensubstanz bezogen, aus gut löslichen Inulooligosacchariden. Der bevorzugte Polymerisatonsgrad liegt zwischen 3 und 7. An Glucose, Fructose und Saccharose sind um 1 %, 0,5 bzw. 5 bis 6 %, auf Trockensubstanz bezogen, im Produkt enthalten. Auf Grund des Inulinaufbaus ergeben sich Oligosaccharide mit gleicher Kettenlänge unterschiedlicher Zusammensetzung. So sind die Ketten einerseits homopolymer und nur aus Fructose aufgebaut. Es existiert ein reduzierendes Ende. Andererseits gibt es heteropolymere Ketten, die neben Fructose am nichtreduzierenden Ende des Gesamtmoleküls ein Saccharosemolekül aufweisen.

Laut Erfindung wurde überraschenderweise auch gefunden, daß ein saccharosefreies Produkt erhalten wird, wenn zu der inulinhaltigen Lösung eine α-Glucosidase, aus Hefe isoliert, hinzugegeben wird. Es ist dabei zweckmäßig, diese enzymatische Behandlung vor der chromatographischen Trennung am Ca-beladenen Kationenaustauscher durchzuführen, da so die aus der Saccharosespaltung entstehenden Kohlenhydrate Glucose und Fructose abgetrennt werden und schließlich ein Endprodukt erhalten wird, das zusammen an Glucose und Fructose weniger als 2 % auf Trockensubstanz enthält.

Die Verwendung der $\alpha$-Glucosidase kann in löslicher Form erfolgen bzw. als immobilisierter Katalysator.

Da die heteropolymeren Anteile der Inulooligosaccharide identisch bezüglich Aufbau und Zusammensetzung mit dem bereits erwähnten Neosugar sind, gelten für diesen Anteil der Inulooligosaccharide, die in der Literatur bereits beschriebenen Eigenschaften, wie z. B. Kalorienreduktion bei monogastrischen Tieren und Menschen.

Um Aussagen über den homopolymeren Anteil der Inulooligosaccharide bezüglich Verwertbarkeit im Dünndarm bei monogastrischen Tieren zu erhalten, sind Spaltungsversuche mit isolierten Enzymkomplexen der Dünndarmmucosa vom Schwein durchgeführt worden. Eine Spaltung der Inulooligosaccharide konnte weder mit dem isolierten Saccharase/Isomaltase-Komplex noch mit dem isolierten Glucoamylase/Maltase-Komplex beobachtet werden. Diese Ergebnisse weisen auf die Verwendung als kalorienreduzierter Füllstoff hin.

Das erfindungsgemäße Verfahren zeichnet sich auch durch die hohen Ausbeuten an Inulooligosacchariden aus, da hier das Pflanzenmaterial direkt mit Endo-Inulinase umgesetzt und auf eine vorgeschaltete Extraktion des Inulins verzichtet wird. Somit sind Verluste von 10 bis 20 %, die bei der Extraktion von Inulin aus Pflanzenmaterial üblicherweise auftreten, nicht gegeben.

### Isolierung der Endo-Inulinase

Die Isolierung der verwendeten Endo-Inulinase erfolgt, in dem Inulinase SP230 (Fa. Novo) nach einem vorangeschalteten Entsalzungsschritt an CM Sepharose CL 6B chromatographiert worden ist. Hierbei wird die Endo-Form der Inulinase nicht am Austauscher gebunden und mit dem Vorlauf von der Säule eluiert.

Die Erfindung wird durch folgende Beispiele näher erläutert:

### Beispiel 1

60 kg vorzerkleinerte Zichorienwurzeln werden nach Zugabe von 120 l Wasser mittels Supratonmaschine in einem kontinuierlichen Verfahren zu einem Brei verarbeitet, dabei wird der Brei auf 95 °C erhitzt und bei dieser Temperatur für 30 Minuten belassen. Nach Abkühlen des Breies auf 56 °C wird der pH-Wert mit 33 %iger $H_2SO_4$ auf pH 5,4 eingestellt. Nach Zugabe von 2 Enzymeinheiten an Endo-Inulinase pro Gramm Inulin wird der Ansatz für 15 Stunden bei 56 °C unter Rühren belassen. Die Konstanthaltung des pH-Wertes auf 5,4 erfolgt durch Zudosierung von Kalkmilch mit einem CaO-Gehalt von 100 g/l.

Die enzymatische Reaktion wird durch Anheben des pH-Wertes auf 10,7 durch Zugabe von $Ca(OH)_2$ unter Rühren gestoppt. Man beläßt den Ansatz für 30 Minuten hei diesem pH-Wert und trennt anschließend den Feststoff mittels Filterkammerpresse ab. Der Feststoff wird mit weiteren 50 l Wasser gewaschen. Die vereinigten Filtrate werden durch Einleiten von Kohlensäure neutralisiert. Es folgt eine Aufkonzentrierung auf 20 % Trockensubstanzgehalt.

Eine Entsalzung und Entfärbung der Lösung wird am schwach sauren Kationenaustauscher in der $H^+$-Form (wie etwa Lewatit® CNP 80, Fa. Bayer AG) und am mittelbasischen Anionenaustauscher in der $OH^-$-Form (wie etwa Lewatit® MP 64, Fa. Bayer AG) durchgeführt. Dazu sind jeweils 10 l Austauscher in eine entsprechende Säule gefüllt. Die Säulen sind so hintereinandergeschaltet, daß die Produktlösung zuerst den Kationenaustauscher passiert, befolgt vom Anionenaustauscher. Dieser Schritt wird bei Zimmertemperatur durchgeführt.

Eine endgültige Reinigung wird an einer Säule, gefüllt mit 5 l gekörnter Aktivkohle erzielt.

Das inulooligosaccharidhaltige Effluat wird auf 20 % Trockensubstanzgehalt aufkonzentriert, steril filtriert und sprühgetrocknet. Erhalten werden 7,85 kg Produkt.

Nach gelchromatographischer Trennung weist das Produkt folgende Zusammensetzung auf:

| | % Fläche |
|---|---|
| Monosaccharide | 5,7 |
| Disaccharide | 10,3 |
| Trisaccharide | 20,3 |
| Tetrasaccharide | 20,3 |
| Pentasaccharide | 16,8 |
| Hexasaccharide | 12,6 |
| Heptasaccharide | 7,5 |
| > Heptasaccharide | 6,5 |
| | 100,0 |

Die Gehalte an Glucose, Fructose und Saccharose, auf Trockensubstanz bezogen, betragen 3,4 %, 2,3 % bzw. 6,5 %.

Beispiel 2

127 kg vorzerkleinerte Zichorien (14,5 % Inulin-Gehalt) werden nach Zugabe von 190 l Wasser, wie in Beispiel 1 besprochen, zu einem Brei zerkleinert und pasteurisiert. Nach Einstellen der Temperatur von 56 °C und des pH-Wertes von 5,4 werden 2,2 Enzymeinheiten an Endo-Inulinase pro g Inulin zugegeben. Die weitere Vorgehensweise einschließlich der Herstellung der Filtratlösung erfolgt wie bei Beispiel 1.

Die Filtrate werden auf 43 % Trockensubstanzgehalt aufkonzentriert. Der filtrierte Sirup wird aufgeteilt und in zwei Chromatographieläufen an einer 500 l - Trennanlage, gefüllt mit einem schwach vernetzten Ca-beladenen Kationenaustauscher (etwa vom Typ Lewatit® MDS 1368, Fa. Bayer AG) bei 60 °C unter einer Flußrate von 100 l Wasser pro Stunde chromatographiert.

Die inulooligosaccharidhaltigen Fraktionen der beiden Chromatographieläufe werden vereint und auf 20 % Trockensubstanzgehalt aufkonzentriert. Es schließt sich eine Entsalzung und Entfärbung der Lösung an Austauschern, wie unter Beispiel 1 beschrieben, an.

Das inulooligosaccharidhaltige Effluat wird auf 20 % Trockensubstanzgehalt aufkonzentriert, steril filtriert und sprühgetrocknet. Erhalten werden 14,6 kg Produkt.

Nach gelchromatographischer Auftrennung weist das Produkt folgende Zusammensetzung auf:

|  | % Fläche |
|---|---|
| Monosaccharide | 1,5 |
| Disaccharide | 8,6 |
| Trisaccharide | 14,7 |
| Tetrasaccharide | 19,5 |
| Pentasaccharide | 21,2 |
| Hexasaccharide | 17,7 |
| Heptasaccharide | 9,8 |
| > Heptasaccharide | 7,0 |
|  | 100,0 |

Die Gehalte an Glucose, Fructose und Saccharose betragen, auf Trockensubstanz bezogen, 0,9 %, 0,6 % bzw. 5,4 %.

Beispiel 3

Untersucht wurde der Einfluß des pH-Wertes auf die Zusammensetzung der Inulooligosaccharide.

Sechs Ansätze sind durchgeführt worden. Als Ausgangsmaterialien werden getrocknete und frische Zichorien sowie frische Topinamburknollen verwendet. Zu 550 g getrockneten Zichorien werden in einem 10 l Reaktionsgefäß (Biostat S) 6 l Wasser gegeben. Im Falle der frischen Pflanzenmatierialien werden je 2,2 kg gewaschene Zichorien bzw. Topinamburknollen mit 4,4 l Wasser gemust. Von jedem Pflanzenmaterial werden zwei parallele Ansätze gemacht:

1. mit pH-Kontrolle bei 5,4
2. ohne pH-Kontrolle.

Die Anfangs-pH-Werte sind jeweils vor Zugabe der Endo-Inulinase (2 Enzymeinheiten pro g Inulin) mit verdünnter $H_2SO_4$ unter Rühren auf 5,4 eingestellt worden. Die pH-Konstanthaltung erfolgte durch Zudosierung von 0,1 N NaOH. Die Ansätze werden nach Enzymzugabe jeweils für 16 Stunden bei 56 °C unter Rühren belassen. Gestoppt wird die enzymatische Reaktion durch Aufheizen der Ansätze für 15 Minuten auf 95 °C.

Die Zusammensetzung der Inulooligosaccharide ist gelchromatographisch ermittelt worden. Die Resultate sind in Tabelle 1 zusammengestellt.

Beispiel 4

Spaltung von Saccharose in inulooligosaccharidhaltige Lösung

Hierzu sind 10 ml Lösung, die wie in Beispiel 2 beschrieben, nach enzymatischer Behandlung durch Endo-Inulinase und dem sich anschließenden Filtrationsschritt erhalten wird, eingesetzt worden. Das Filtrat mit einem Trockensubstanzgehalt von 7,8 % enthält 12,5 % an Disacchariden. Davon sind 66 % Saccharose und 34 % Inulobiose. Die Lösung wird mit 0,1 NaOH auf pH 7 eingestellt und auf 37 °C erwärmt. Anschließend wird 0,3 ml α-Glucosidase (Fa. Boehringer, Mannheim) hinzugegeben. Nach 8 Stunden wird die enzymatische Reaktion durch Erhitzen auf 95 °C für 15 Minuten gestoppt.

Ergebnis: Die Saccharose ist nicht mehr nachzuweisen.

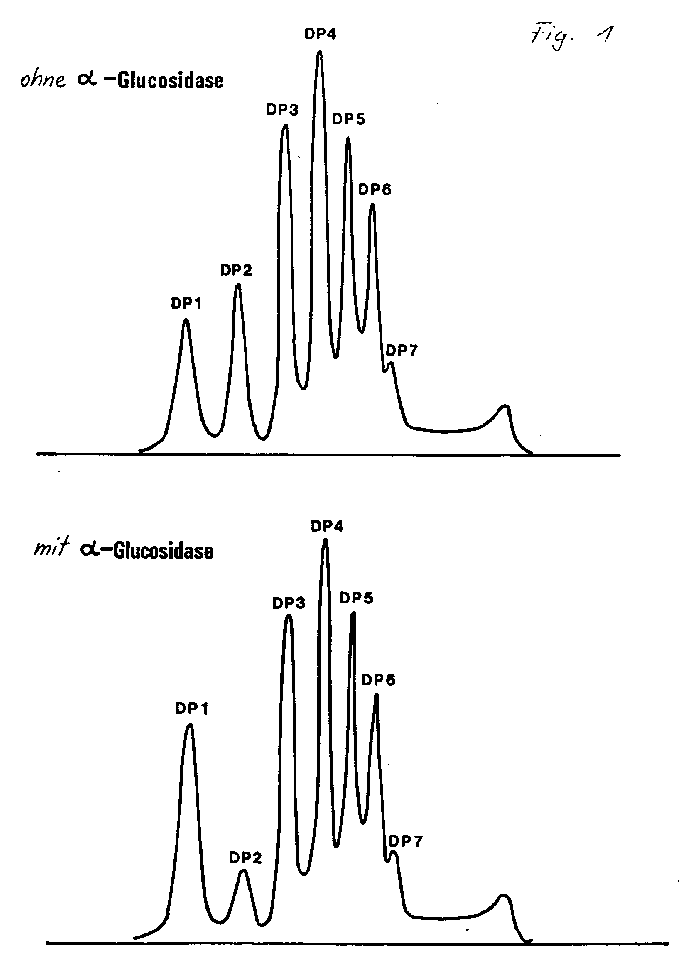

Die gelchromatographische Trennung (Fig. 1) zeigt eine Verringerung des Disaccharid-Anteiles und einen Anstieg bei den Monosacchariden. Die Inulooligosaccharid-Zusammensetzung verändert sich nicht.

Beispiel 5

Die Bestimmung der Enzymaktivität erfolgt mit einer 4 %-igen Inulinlösung. Hierfür wird das Inulin in 0,1 M Natrium-acetatpuffer, pH 5,0 gelöst. Die Reaktion selbst wird bei 50 °C durchgeführt und exakt nach 10 Minuten gestoppt. Dieses geschieht durch Zugabe von Dinitrosalicylsäure-Lösung. Der Nachweis erfolgt nach Erhitzen der Lösung und anschließendem Messen der Extinktion bei $\lambda = 546$ nm gegen den Leerwert. Eine Enzymeinheit an Endoinulinase ist die Enzymmenge, die 1 $\mu$Mol reduzierenden Zucker pro Minute unter obigen Bedingungen bildet.

EP 0 440 074 B1

Tabelle 1

| | Zichorien getrocknet | | Zichorien frisch | | Topinambur frisch | |
|---|---|---|---|---|---|---|
| | pH variabel | konstant | pH variabel | konstant | pH variabel | konstant |
| Anfangs-pH-Wert | 5,4 | 5,4 | 5,4 | 5,4 | 5,4 | 5,4 |
| End-pH-Wert | 4,5 | 5,4 | 4,7 | 5,3 | 4,8 | 5,4 |
| **% Fläche** | | | | | | |
| Monosaccharide | 5,4 | 1,7 | 17,0 | 13,3 | 24,8 | 8,5 |
| Disaccharide | 11,7 | 15,0 | 6,5 | 8,5 | 17,3 | 23,2 |
| Trisaccharide . | 20,9 | 19,2 | 20,8 | 21,0 | 18,3 | 21,0 |
| Tetrasaccharide | 18,3 | 15,6 | 19,5 | 19,7 | 16,3 | 17,0 |
| Pentasaccharide | 14,0 | 13,8 | 14,7 | 14,6 | 12,4 | 13,4 |
| Hexasaccharide | 11,2 | 11,0 | 12,0 | 12,6 | 8,4 | 9,9 |
| Heptasaccharide | 6,9 | 8,2 | 5,2 | 5,9 | 2,5 | 3,2 |
| > Heptasaccharide | 11,6 | 15,5 | 4,3 | 4,4 | - | 3,8 |

**Patentansprüche**

1. Verfahren zur Herstellung eines glucose-, fructose- und saccharosearmen Inulooligosaccharid-Produktes, wobei man das Inulinhaltige Pflanzenmaterial direkt nach Zerkleinerung und Pasteurisierung einer enzymatischen Behand- lung mit Endo-Inulinase bei Temperaturen von 50- 60 °C unterzieht, **dadurch gekennzeichnet,** daß

   a) der pH-Wert bei der enzymatischen Behandlung bei 5,0 - 5,6 konstant gehalten wird

   b) die entstandene Saccharose durch Zugabe von α-Glucosidase entfernt wird und

   c) Glucose und Fructose chromatographisch abgetrennt werden.

2. Verfahren nach Anspruch 1, **dadurch gekennzeichnet,** daß der pH-Wert während der enzymatischen Behandlung mit Endo-Inulinase konstant gehalten wird und zwischen 5,3 und 5,4 liegt.

3. Verfahren nach Anspruch 1 bis 2, dadurch gekennzeichnet, daß 1,8 bis 2,4 Enzymeinheiten pro Gramm Inulin an Endo-Inulinase eingesetzt werden.

4. Verfahren nach Anspruch 1 bis 3, dadurch gekennzeichnet, daß die Temperatur bei der enzymatischen Behandlung mit Endoinulinase bei 56 °C liegt.

5. Verfahren nach Anspruch 1 bis 4, dadurch gekennzeichnet, daß die irreversible Inaktivierung der Endo-Inulinase durch Anheben des pH-Wertes auf 10,7 bis 11,0 mittels $Ca(OH)_2$ erfolgt.

6. Verfahren nach Anspruch 1 bis 5, dadurch gekennzeichnet, daß die erhaltene wäßrige Phase auf einen Trockensubstanzgehalt von 40 - 60 % eingedampft und anschließend über einen stark sauren, insbesondere schwach vernetzten Kationenaustauscher in der Ca-Form getrennt wird, um so störende Glucose und Fructose sowie hochmolekulare Anteile von der inulooligosaccharidhaltigen Fraktion abzutrennen.

7. Verfahren nach Anspruch 1 bis 6, dadurch gekennzeichnet, daß vor der chromatographischen Trennung am stark sauren, insbesondere schwach vernetzten Kationenaustauscher in der Ca-Form eine Behandlung des Filtrates mit α-Glucosidase erfolgt.

8. Verfahren nach Anspruch 7, dadurch gekennzeichnet, daß die α-Glucosidasebehandlung mit Enzymlösung bzw. immobilisierter α-Glucosidase in einem pH-Bereich von 6,5 - 7,2 bei einer Temperatur von 33 - 50 °C erfolgt.

**Claims**

1. Process for preparing a low glucose, fructose and saccharose inulooligosaccharide product, in which vegetable material containing inulin is subjected to enzymatic treatment with endoinulinase at temperatures of 50 to 60°C directly after crushing and pasteurization, characterised in that

   a) the pH value is kept constant at 5.0 - 5.6 during the enzymatic treatment;

   b) the resulting sucrose is removed by the addition of α-glucosidase, and

   c) glucose and fructose are isolated by means of chromatography.

2. Process according to claim 1, characterised in that the pH value is kept constant during the enzymatic treatment with endoinulinase and is between 5.3 and 5.4.

3. Process according to claims 1 to 2, characterised in that 1.8 to 2.4 enzyme units of endoinulinase are used per gram of inulin.

4. Process according to claims 1 to 3, characterised in that the temperature during the enzymatic treatment with endoinulinase is 56°C.

5. Process according to claims 1 to 4, characterised in that the endoinulinase is irreversibly inactivated by raising the pH value to 10.7 to 11.0 by means of $Ca(OH)_2$.

6. Process according to claims 1 to 5, characterised in that the aqueous phase obtained is concentrated to a dry substance content of 40 - 60% and subsequently separated by way of a strongly acidic, in particular slightly cross-linked cationic exchanger in Ca form in order to isolate interfering glucose and fructose as well as high-molecular proportions of the fraction containing inulooligosaccharide.

7. Process according to claims 1 to 6, characterised in that, before chromatographic separation at the strongly acidic, in particular slightly cross-linked cationic exchanger in Ca form, treatment of the filtrate with a-glucosidase takes place.

8. Process according to claim 7, characterised in that the $\alpha$-glucosidase treatment with an enzyme solution or with immobilised a-glucosidase takes place within a pH range of 6.5 - 7.2 at a temperature of 33 - 50°C.

## Revendications

1. Procédé de fabrication d'un produit inulo-oligo-saccharide pauvre en glucose, fructose et saccharose, dans lequel on soumet la matière végétale contenant de l'inuline, directement après broyage et pasteurisation, à un traitement enzymatique à l'endo-inulinase à des températures de 50 à 60°C,
   **caractérisé** en ce que :
   a) le pH est maintenu constant à 5,0 - 5,6 pendant le traitement enzymatique,
   b) le saccharose formé est éliminé par une addition de $\alpha$-glucosidase,
   et
   c) le glucose et le fructose sont séparés par chromatographie.

2. Procédé selon la revendication 1, caractérisé en ce que le pH est maintenu constant pendant le traitement enzymatique à l'endo-inulinase et se situe entre 5,3 et 5,4.

3. Procédé selon les revendications 1 et 2, caractérisé en ce qu'on met en oeuvre 1,8 à 2,4 unités d'enzyme d'endo-inulinase par gramme d'inuline.

4. Procédé selon les revendications 1 à 3, caractérisé en ce que la température lors du traitement enzymatique à l'endo-inulinase est de 56°C.

5. Procédé selon les revendications 1 à 4, caractérisé en ce que l'inactivation irréversible de l'endo-inulinase a lieu par relèvement du pH à une valeur de 10,7 à 11,0 au moyen de $Ca(OH)_2$.

6. Procédé selon les revendications 1 à 5, caractérisé en ce que la phase aqueuse obtenue est concentrée par évaporation jusqu'à une teneur en matiére sèche de 40 à 60% et est ensuite séparée sur un échangeur de cations fortement acide, en particulier faiblement réticulé, sous la forme Ca, pour séparer ainsi de la fraction contenant l'inulo-oligo-saccharide le glucose et le fructose importuns ainsi que les parties de poids moléculaire élevé.

7. Procédé selon les revendications 1 à 6, caractérisé en ce qu'un traitement du filtrat à l'$\alpha$-glucosidase a lieu avant la séparation chromatographique sur l'échangaur de cations fortement acide, en particulier faiblement réticulé, sous la forme Ca.

8. Procédé selon la revendication 7, caractérisé en ce que le traitement à l'$\alpha$-glucosidase a lieu avec une solution de l'enzyme ou avec de l'$\alpha$-glucosidase immobilisée dans un domaine de pH de 6,5 à 7,2 à une température de 33 à 50°C.

Fig. 1

ohne α –Glucosidase

mit α–Glucosidase